# EUROPÄISCHE PATENTSCHRIFT

(11) **EP 0 524 554 B1**
(45) Veröffentlichungstag und Bekanntmachung des Hinweises auf die Patenterteilung: **29.10.1997**
(21) Anmeldenummer: 92112212.3
(22) Anmeldetag: 17.07.1992
(51) Int. Cl.: C07C 263/04, B01J 6/00

(54) **Verfahren zur thermischen Spaltung von Carbamidsäureestern**
Process for thermal cleavage of carbamic acid esters
Procédé pour la dissociation thermique des esters d'acides carbamiques

(30) Priorität: 25.07.1991 DE 4124671
(43) Veröffentlichungstag der Anmeldung: 27.01.1993
(73) Patentinhaber: BASF Aktiengesellschaft, 67063 Ludwigshafen (DE)
(72) Erfinder: Schwarz, Hans Volkmar, Dr., Baton Rouge, LA (US); Otterbach, Andreas, Dr., W-6710 Frankenthal (DE); Mattner, Otto, W-6720 Speyer (DE); Merger, Franz, Dr., W-6710 Frankenthal (DE); Schwarz, Wolfgang, Dr., W-6701 Otterstadt (DE); Brandt, Eckhardt, Dr., W-6707 Schifferstadt (DE); Magnussen, Peter, Dr., W-6702 Bad Duerkheim (DE); Minges, Roland, Dr., W-6718 Grünstadt (DE)

(56) Entgegenhaltungen:
- EP-A- 0 054 817
- EP-A- 0 092 738
- EP-A- 0 126 299

## Beschreibung

Gegenstand der vorliegenden Erfindung ist ein Verfahren zur thermischen Spaltung von mono- und polyfunktionellen Carbamidsäureestern in die korrespondierenden Isocyanate und die Hydroxylkomponente in der Flüssigphase.

Reaktoren zur Spaltung von Carbamidsäureestern zu Isocyanaten sind in verschiedenen Patentschriften beschrieben. Die Spaltung der Carbamidsäureester nach Verdampfung in der Gasphase wird beispielsweise in den europäischen Patentschriften 28 724, 126 299, 126 300, 100 047 und in den US-Patentschriften 3 734 941 und 3 870 739 beschrieben. Nachteilig ist hierbei die Verstopfungsgefahr im vorgeschalteten Verdampfer, die eine technische Anwendung erschwert.

Die Spaltung in einem Wirbelbett oder Rührbett aus Kohlenstoffpulver nach Verdüsen des Carbamidsäureesters in dieses Bett hinein, beschrieben in der EP 78 005, liefert zwar gute Ausbeuten, läßt jedoch Schwierigkeiten bei der technischen Realisierung erwarten.

Die Spaltung in der Flüssigkeitsphase macht es erforderlich, mindestens eines der beiden Spaltprodukte, also das Isocyanat oder die Hydroxylkomponente, sofort aus der Reaktionslösung gasförmig zu entnehmen, um eine Rückreaktion zu verhindern. Zweckmäßigerweise werden beide Spaltprodukte gasförmig entnommen und später getrennt. Wegen der Bildung höher-molekularer irreversibler Nebenprodukte ist es erforderlich, einen Teil des Reaktionsmediums kontinuierlich aus dem Reaktor zu entnehmen und damit die Nebenprodukte auszuschleusen.

Um Nebenreaktionen zu irreversiblen Zersetzungsprodukten zurückzudrängen ist es sinnvoll, die Reaktionstemperatur durch Zuhilfenahme von Katalysatoren abzusenken. Basische Katalysatoren, beschrieben in den US-Patentschriften 2 692 275 und 2 713 591, beschleunigen jedoch auch die Zersetzungsreaktionen. Besser geeignete Katalysatoren - für die Spaltung aromatischer Carbamidsäureester - werden in der DE-S 26 35 490 beschrieben. Aluminium-, Zink- und Zinnverbindungen dominieren. Sie werden in Konzentrationen deutlich unter 1 % eingesetzt und beschleunigen bevorzugt die Spaltungsreaktion.

Für die Herstellung mehrfunktioneller Isocyanate, bei denen die Gefahr der Polymerisation und Rückstandsbildung verstärkt besteht, wurde die Verdünnung der Carbamidsäureester mit inerten Lösungsmitteln, beschrieben in den DE-AS 24 21 503 und 25 26 193, oder das Zudosieren von inerten Lösungsmitteln in den Spaltreaktor, beschrieben in der EP 92 738, vorgeschlagen, um Belegungen der Flächen im Reaktor zurückzudrängen und Nebenprodukte auszuschleusen. Nachteilig an einem solchen Zusatz von Hilfsmitteln sind der daraus resultierende erhöhte verfahrenstechnische Aufwand bzw. die Kosten für deren Verbrauch.

In der US-Patentschrift 4 294 774 wird die Spaltung in katalytisch wirksamen Lösungsmitteln, Dialkylanilinen, vorgeschlagen.

In der EP 92 738 wird jedoch die lösungsmittelfreie Spaltung nicht ausgeschlossen. Als geeignete Reaktoren werden Dünnschichtverdampfer und Fallfilmverdampfer genannt. Nachteilig an diesen Reaktoren sind bewegte Teile beim bevorzugten Dünnschichtverdampfer, die hohen Kosten derartiger Reaktoren im technischen Maßstab und als wichtigster Nachteil die Anfälligkeit solcher Reaktoren gegen Verstopfung durch Polymerisation bei der Spaltung mehrfunktioneller Carbamidsäureester, wenn die Reaktoren über längere Zeit als einige Tage kontinuierlich betrieben werden, was für einen wirtschaftlichen technischen Reaktor unumgänglich ist. Die beschriebenen Reaktoren weisen alle somit Mängel hinsichtlich ihrer technischen Verwendbarkeit auf.

Aufgabe der vorliegenden Erfindung war es, ein Verfahren zu finden, in dem die katalysierte oder unkatalysierte Spaltung von Carbamidsäureestern ohne Verwendung eines Löse- oder Verdünnungsmittels, kontinuierlich über technisch sinnvolle Zeiträume durchgeführt werden kann, ohne daß der Betrieb wegen Verstopfung oder Fouling durch polymere Nebenprodukte unterbrochen werden muß.

Ferner mußte eine Methode zur Abtrennung der Spaltgase aus dem Reaktor und zu ihrer Trennung gefunden werden, die ebenfalls frei von Verstopfungs- und Foulingproblemen ist. Zudem mußte die Anwesenheit von Nebenprodukten, wie sie in einem Kreislaufverfahren zur Erzeugung von Isocyanaten aus Aminen über die Zwischenstufe der Carbamidsäureester auftreten können, im Reaktor möglich sein, ohne daß hierdurch die genannten Probleme wiederum auftreten.

Diese Aufgabe wird erfindungsgemäß dadurch gelöst, daß man das erfindungsgemäße Verfahren in einem Reaktor durchführt, der Einbauten zur Einbringung der Reaktionswärme enthält und dessen Geometrie, gekennzeichnet durch die Entgasungsfläche im Verhältnis zum Volumen und die Anordnung der Heizflächen, die Spaltung in einem zweiphasigen Gemisch bei einem volumetrischen Gasgehalt von über 50 % erlaubt.

Weitere Merkmale des erfindungsgemäßen Verfahrens sind Gegenstand der Unteransprüche.

Überraschenderweise wurde gefunden, daß sich Carbamidsäureester, insbesondere auch bifunktionelle und mehrfunktionelle Carbamidsäureester nicht nur in technisch anspruchsvollen Apparaten, wie Fallfilmverdampfern, Dünnschichtverdampfern und Rührreaktoren, sondern auch in einfachen, im folgenden näher beschriebenen Reaktoren ohne bewegte Teile mit hohen Standzeiten der Reaktoren und guten Ausbeuten spalten lassen, ohne daß Löse- oder Verdünnungsmittel eingesetzt werden müssen. Überraschend war insbesondere, daß Standzeiten von mehreren Monaten kontinuierlichen Betriebes erreicht werden konnten, ohne daß der Reaktor wegen Belegungen mit polymeren Nebenprodukten abgestellt werden mußte.

Als Reaktoren sind Kessel geeignet, die Einbauten zur Einbringung der Reaktionswärme enthalten. Das Reaktionsmedium liegt darin in einem siedeähnlichen Zustand vor, der dadurch gekennzeichnet ist, daß die aus der Spaltreaktion resultierenden Gase und Dämpfe des Edukts gebildet werden und in der Flüssigkeit aufsteigen.

Die Resistenz gegen polymere Belegungen wird durch die spezielle Geometrie der Reaktoren und das Betreiben der Reaktoren in einem technisch bisher nicht üblichen Betriebszustand erreicht: Der Reaktor wird so betrieben, daß die zweiphasige Mischung aus siedender Flüssigkeit und Dampfphase, die aus dem verdampften Edukt und den Spaltgasen besteht, über 50 % volumetrischen Gasanteil enthält. Die Reaktion wird demnach bei einem Strömungszustand durchgeführt, wie er bisher bei technischen Verdampfern nicht üblich ist. Üblich sind über 70 % Flüssigkeit, bzw. weniger als 30 % Gasgehalt im verdampfenden Medium in der beheizten Verdampferzone. Gasgehalte von über 30 % ("scheinbarer Flüssigkeitsstand" von unter 70 %) führt in technisch üblichen Verdampfern zu schlechterem Wäremübergang und anderen Nachteilen wie z.B. Abreißen des Naturumlaufs in Robertverdampfern. Zusammenhänge von Verdampfereigenschaften mit dem volumetrischen Gasgehalt des Mediums können z.B. nachgelesen werden in "Strömung und Wärmeübergang in Gas-Flüssigkeits-Gemischen", Franz Mayinger, Springer Verlag, 1982, Kapitel 1.3 und 2.4 und in der Habilitationsschrift "Partielles Filmsieden in Zweiphasenströmungen" von Dr. Ing. habil. Hein Auracher, Fortschritt-Berichte VDI, Reihe 3 "Verfahrenstechnik" Nr. 142, VDI-Verlag 1987 sowie in "Evaporation Technology" von Reinhard Billet, Verlag Chemie, Weinheim, 1989, S. 114-119.

Somit wird bei dieser Betriebsweise über den bisher üblichen Stand der Technik hinausgegangen.

Der volumetrische Gasgehalt der zweiphasigen Mischung soll zwischen 50 und 98 %, vorzugsweise zwischen 60 und 96 % und insbesondere bevorzugt zwischen 75 und 90 % liegen.

Zur Realisierung derart hoher Gasgehalte in der zweiphasigen Mischung sind bestimmte geometrische Anforderungen an die Reaktoren sinnvoll.

So ist es beispielsweise sinnvoll, die Höhe der Reaktionszone im Reaktor nicht kleiner als 0,2 m und nicht größer als 2 m zu wählen, um die Ausbildung der gasreichen zweiphasigen Mischung zu fördern. Die Entgasungsfläche des Reaktors sollte so bemessen sein, daß die Gasgeschwindigkeit nicht unter 1 m/s und nicht über 30 m/s beträgt. Sie beträgt bevorzugt 1 bis 30 m/s, besonders bevorzugt 2 bis 20 m/s, wenn man die Reaktion bei einem Absolutdruck von 2 bis 200 mbar durchführt. Die Wärmeübertragungsflächen sollten so dimensioniert sein, daß die Temperaturdifferenz zwischen Heiz- und Reaktionsmedium kleiner als 40°C ist, aber gleichzeitig die für die stark endotherme Reaktion erforderliche Wärmemenge in das durch Verweilzeitanforderungen beschränkte Volumen eingebracht werden kann.

Gemäß dieser Anforderungen kommen als Spaltreaktoren in Frage: Robertverdampfer, Herbertverdampfer mit und ohne Zwangsumlauf, "Heizkerzenverdampfer" mit senkrechten, schrägstehenden oder waagrechten Heizkerzen, Caddletype Verdampfer, Kessel mit eng gewickelten Heizschlangen, und ähnliche Reaktoren, die sich durch einen hohen Wärmeeintrag in ein kleines Volumen auszeichnen. Die Reaktoren werden voll-kontinuierlich betrieben sowohl hinsichtlich der Produktzufuhr als auch hinsichtlich einer Entnahme von Flüssigkeit, um die Anreicherung höher siedender Nebenprodukte zu vermeiden. Der Umsatz im Reaktor pro Durchgang beträgt zwischen 30 und 95, vorzugsweise zwischen 60 und 90 % Umwandlung von Urethan im Reaktorzulauf in entnommenes Isocyanat. Die Verweilzeit der siedenden Flüssigkeit im Reaktor beträgt mehrere Minuten und kann nicht als "sehr kurz" bezeichnet werden, was einen wesentlichen Unterschied zu den in EP 92 738 genannten Dünnschichtreaktoren darstellt.

Bevorzugt verwendet man einen Heizkerzenreaktor, der senkrecht stehende Heizkerzen in einem Hohlraum, bevorzugt einem zylindrischen Hohlraum oder einem sich nach oben erweiternden Hohlraum enthält, wobei die Heizfläche der Heizkerzen das 5- bis 100fache, bevorzugt das 10- bis 50fache der freien Entgasungsfläche beträgt, und die freie Entgasungsfläche als die Querschnittsfläche des Reaktionshohlraums am oberen Ende der Reaktionszone abzüglich der durch die Kerzen eingenommenen Fläche definiert ist. Gleichzeitig beträgt das Verhältnis Heizfläche pro Reaktionsvolumen zwischen 10 und 320 m²/m³, bevorzugt 20 bis 150 m²/m³.

Gut eignen sich weiterhin Robertverdampfer, Herbertverdampfer, Caddletype Verdampfer, Rohrbündelreaktoren, Kessel mit eng gewickelter innenliegender Heizschlange oder andere bulk-Reaktoren, die bezüglich der Reaktionsführung einen scheinbaren Flüssigkeitsstand von < 50 % erlauben und welche die erfindungsgemäßen Gasgehalte bzw. Gasgeschwindigkeiten ermöglichen.

Die Verweilzeiten betragen 1-60 min, bevorzugt 5-30 min, besonders bevorzugt 5 bis 25 min, und sind definiert als der Quotient aus dem flüssigen Inhalt des Reaktors unter Reaktionsbedingungen und dem pro Minute aus dem Reaktor flüssig entnommenen Reaktorinhalt.

Die Reaktion wird ohne jeden Zusatz von Löse- oder Verdünnungsmittel durchgeführt und ohne daß ein Inertgas durch den Reaktor gefahren wird. Dies steht im Gegensatz zu EP 92 738, wo Verdünnungsmittel ausdrücklich zugelassen sind.

Es ist für die Erfindung wesentlich, daß sich die genannten Reaktoren bei Einhaltung der beschriebenen Randbedingungen so betreiben lassen, daß auch mehrfunktionelle Carbamidsäureester zu mehrfunktionellen Isocyanaten und der jeweiligen Hydroxylkomponente gespalten werden können, ohne daß die Reaktoren im Verlauf einer mehrwöchigen kontinuierlichen Betriebszeit abgestellt und gereinigt werden müssen.

Somit ist im Unterschied zu vorbeschriebenen Reaktoren die Voraussetzung für den kontinuierlichen technischen Betrieb derartiger Reaktoren gegeben.

Der Druck im Reaktor beträgt bevorzugt 1 bis 500 mbar, besonders bevorzugt 5 bis 100 mbar.

Die Rektifikation wird insbesondere bei einem Druck von kleiner als 500 mbar, bevorzugt kleiner als 100 mbar, durchgeführt.

### Definition des Reaktorzulaufs

In den Reaktor wird ein Produktgemisch gefahren, das aus dem Carbamidsäureester und aus Nebenprodukten besteht, die in einem Kreislaufverfahren entstehen, das dadurch gekennzeichnet ist, daß aus einem Amin unter Zuhilfenahme von Harnstoff und einer Hydroxylkomponente ein Carbamidsäureester hergestellt wird, der dann zum Isocyanat gespalten wird, wobei ein Teil des Reaktorinhalts dem Reaktor entnommen und wieder in die erste Stufe des Verfahrens zurückgefahren wird. Solche Nebenprodukte können unter anderem hochmolekulare Produkte, vorzugsweise Isocyanurate, sein. Überraschenderweise genügt es, wenn der Gehalt an Carbamidsäureester im Reaktorzulauf zwischen 80 und 90 % beträgt. Eine höhere Reinheit des Carbamidsäureesters erleichtert den Betrieb des Spaltreaktors, da dann Foulingprobleme an Bedeutung verlieren. Es können auch Reaktorzuläufe mit weniger als 80 % Carbamidsäureester verwendet werden, aber Foulingprobleme nehmen dann zu, insbesondere wenn mehrfunktionelle Carbamidsäureester gespalten werden. Der Spaltreaktorzulauf kann gegebenenfalls den für die Spaltung eingesetzten Katalysator enthalten.

Wesentlich für die Erfindung ist die Tatsache, daß der Carbamidsäureester, bevor er dem Reaktor zugeführt wird, nicht komplett verdampft und wieder kondensiert werden muß, um ihn von den oligomeren Nebenprodukten zu befreien, wie es in der EP 355 443-A2 beschrieben wird, sondern daß ein gewisser Pegel an Nebenprodukt im Reaktor toleriert werden kann.

### Trennaufarbeitung der dem Reaktor entweichenden Gase:

Die dem Reaktor entweichenden Gase bestehen aus den Spaltgasen, d.h. Isocyanat und Hydroxylkomponente, aus verdampftem Einsatzstoff und aus gasförmigen Nebenprodukten.

Im Unterschied zu den EPs 54 817 und 92 738 sowie 355 443 werden die Spaltgase nicht fraktioniert kondensiert an mehreren aufeinanderfolgenden Dephlegmatoren sondern in einer direkt dem Spaltreaktor nachgeschalteten Destillationskolonne rektifiziert. Überraschenderweise trat die in EP 54 817 beschriebene Komplikation, daß die Spaltprodukte bei Verwendung einer Rektifizierkolonne an Stelle von Dephlegmatoren zur Abtrennung der Spaltgase in der Kolonne rekombinieren und kein oder nur wenig Spaltprodukt erhalten wird, nicht auf. Im Gegenteil zur beschriebenen Komplikation ließ sich sogar eine besonders gute Trennwirkung erzielen, sowohl hinsichtlich der Aufgabe nicht abreagierten Carbamidsäureester im Reaktor zurückzuhalten, als auch hinsichtlich der Aufgabe je einen hydroxylkomponenten-reichen und einen isocyanatreichen Produktstrom aus der Kolonne zu erhalten.

Die wesentliche, der Erfindung zugrundeliegende Beobachtung liegt also darin, daß sich die Trennung der dem Reaktor entweichenden Gase in das Edukt Carbamidsäureester und in die Produkte Hydroxylkomponente und Isocyanat in einer dem Reaktor direkt nachgeschalteten Rektifizierkolonne durchführen läßt, die über mindestens einen Rücklauf am Kolonnenkopf verfügt und entweder eine Entnahme eines gasförmigen Produktstromes am Kolonnenkopf enthält, der in nachgeschalteten Trenneinrichtungen weiter aufgearbeitet wird oder eine Entnahme eines der beiden Produkte an einem Seitenabzug der Kolonne und eine Entnahme des anderen Produktes am Kolonnenkopf enthält.

### Weiterverarbeitung des flüssigen Reaktoraustrags

Der flüssige Reaktoraustrag besteht aus nicht umgesetztem Carbamidsäureester, und, im Falle der Spaltung von mehrfunktionellen Carbamidsäureestern, aus teilumgesetztem Ausgangsprodukt, sowie aus Komponenten wie sie schon bei der Beschreibung des Reaktorzulaufs genannt wurden, allerdings in anderer prozentualer Zusammensetzung. In der Regel besteht der Reaktoraustrag zu weniger als einem Drittel aus nicht umgesetztem Edukt. Bevor der Reaktoraustrag in die erste Verfahrensstufe recyclisiert wird, kann er mit der Hydroxylkomponente aus der Spaltungsreaktion unter anderem zwecks Absättigung freier Isocyanatgruppen zur Reaktion gebracht und durch Destillation oder eine andere geeignete Trennoperation von unerwünschten Nebenprodukten befreit werden.

### Beispiel

3,6 kg/h einer Mischung von 83 Gew.-% Hexamethylendi-n-butylurethan-1,6, im folgenden kurz "HDU" genannt, ca. 15 Gew.-% oligomerer Isocyanurate, und kleineren Anteilen anderer Nebenprodukte, wird zusammen mit 0,01 mol-% Dibutylzinndilaurat bezogen auf HDU-kontinuierlich in einen Heizkerzenreaktor gefahren, der 4 dampfbeheizte Heizkerzen von 14 mm Durchmesser und 50 cm Länge in einem zylindrischen Raum von ca. 6,8 cm Durchmesser und 52 cm Länge enthält. Am Boden des Reaktors befindet sich zwischen den Heizkerzen eine Austrittsöffnung für flüssigen Reaktorinhalt von 12 mm Durchmesser. Über der Reaktionszone erweitert sich der Reaktor zu einer Haube von 20 cm Durchmesser und 30 cm Höhe.

Die Spaltreaktion wird bei 240°C im Reaktionsmedium und 30 mbar Druck durchgeführt. Die Dampftemperatur an den Heizkerzen beträgt ca. 260°C.

Der volumetrische Gasgehalt der Reaktionsmischung beträgt ca. 84 %, d.h. die den ganzen Reaktionsraum von ca. 1,6 l ausfüllende zweiphasige Mischung enthält ca. 250 ml Flüssigkeit und ca. 1,35 l Gas- bzw. Dampfanteil.

Am Fuß des Reaktors werden ca. 1 kg/h Reaktorablauf entnommen, der aus 25 Gew.-% HDU, 9 Gew.-% Isocyanatohexamethylen-n-butylurethan (HMI) und ca. 66 Gew.-% höhermolekularen Nebenprodukten, vorzugsweise Isocyanuraten und Allophanaten besteht. Außerdem enthält der Reaktorablauf das gesamte Zinn aus dem Reaktorzulauf. Die Verweilzeit des Reaktionsmediums im Reaktor beträgt somit ca. 15 min.

Die Spaltgase werden in einer mit einer Sulzerpackung gefüllten Rektifizierkolonne getrennt, die sich direkt auf der erwähnten Haube über dem Reaktor befindet. Im unteren Teil der Kolonne werden HDU und HMI abgetrennt und fließen in den Reaktor zurück. 80 cm über dem Reaktor befindet sich ein Seitenabzug, an dem ca. 1,2 kg/h Hexamethylendiisocyanat-1,6 (HDI) in einer Reinheit von ca. 98 Gew.-% entnommen wird. 40 cm über dem Seitenabzug befindet sich ein Kopfkondensator, an dem ca. 1,4 kg/h eines butanolreichen Kopfabzugs entnommen und ein Rücklauf erzeugt wird. Das am Kolonnenkopf entnommene butanolreiche Produkt fällt mit einer Reinheit von über 99 Gew.-% an. Der molare Umsatz von HDU im Reaktorzulauf zu entnommenem HDI beträgt somit in einem Reaktordurchgang ca. 76 %.

Der Reaktorablauf und das abgespaltene Butanol werden in einen Rührkessel gefördert und dort bei 100°C zur Reaktion gebracht. Die resultierende Mischung wird in die Urethanbildungsstufe eines Kreislaufverfahrens zurückgefahren. In der Urethanbildungsstufe wird aus Hexamethylendiamin (HMD), Harnstoff und n-Butanol der Carbamidsäureester HDU hergestellt, der dann nach mehreren Reinigungsschritten in Form der nebenprodukthaltigen Mischung dem Spaltreaktor zugeführt wird.

Das am Seitenabzug der Rektifikationskolonne erhaltene Roh-HDI wird in geeigneten Reinigungsstufen auf marktfähige Reinheit gebracht. Die Ausbeute an HDI, bezogen auf Hexamethylendiamin, betrug im Kreislaufverfahren 93 %, bezogen auf Harnstoff 89 %.

Der Spaltreaktor wies nach 8 Wochen ununterbrochenen Betriebs nur geringe Belegungen auf den Heizflächen auf und mußte während dieser Zeit nie gereinigt werden.

## Patentansprüche

1. Verfahren zur thermischen Spaltung von mono- und polyfunktionellen Carbamidsäureestern in die korrespondierenden Isocyanate und die Hydroxylkomponente in der Flüssigphase, dadurch gekennzeichnet, daß man die Reaktion in einem Reaktor durchgeführt, der Einbauten zur Einbringung der Reaktionswärme enthält und dessen Geometrie, gekennzeichnet durch die Entgasungsfläche im Verhältnis zum Volumen, die Spaltung in einem zweiphasigen Gemisch bei einem volumetrischen Gasgehalt von über 50 % erlaubt.

2. Verfahren nach Anspruch 1, dadurch gekennzeichnet, daß die Geschwindigkeit des entweichenden Gases am oberen Ende der Reaktionszone 1 bis 30 m/s beträgt, wobei man die Reaktion bei einem Absolutdruck von 2 bis 200 mbar durchführt.

3. Verfahren nach Anspruch 1 bis 2, dadurch gekennzeichnet, daß man die Verweilzeit des Reaktionsmediums, bezogen auf den flüssigen Reaktoraustrag, bei 1 bis 60 min einstellt.

4. Verfahren nach den Ansprüchen 1 bis 3, dadurch gekennzeichnet, daß man zur Abtrennung der Spaltgase die dem Reaktor entnommenen Produkte einer unmittelbar nachgeschalteten Rektifikationskolonne zuführt, wobei außer dem Kopfabzug mittels einer weiteren Entnahmestelle gasförmiges oder flüssiges Produkt oder Produktgemisch abgezogen wird, wobei entsprechend der Reihenfolge der Siedepunkte das eine Produkt isocyanatreich und das andere Produkt hydroxylkomponentenreich ist.

5. Verfahren zur Spaltung von Carbamidsäureestern in einem Verfahren nach den Ansprüchen 1 bis 4, dadurch gekennzeichnet, daß man dem Reaktionsmedium kein Lösungsmittel zusetzt.

6. Verfahren zur Spaltung von Carbamidsäureestern in einem Verfahren nach den Ansprüchen 1 bis 5, dadurch gekennzeichnet, daß der Umsatz bei einem Durchgang von Urethan zu Isocyanat mindestens 50 % beträgt.

7. Verfahren zur Spaltung von Carbamidsäureestern in einem Verfahren nach den Ansprüchen 1 oder 3 bis 5, dadurch gekennzeichnet, daß der Druck im Reaktor 1 mbar bis 500 mbar beträgt.

8. Verfahren nach Anspruch 4, dadurch gekennzeichnet, daß man bei einem Druck kleiner als 500 mbar rektifiziert.

## Claims

1. A process for the thermal cleavage of mono- and polyfunctional carbamic esters into the corresponding isocyanates and the hydroxyl component in the liquid phase, wherein the reaction is carried out in a reactor which contains baffles for introducing the heat of reaction and whose geometry, characterized by the degassing area in relation to the volume, permits cleavage in a two-phase mixture at a volumetric gas content of more than 50%.

2. A process as claimed in claim 1, wherein the velocity of the escaping gas at the upper end of the reaction zone is from 1 to 30 m/s, the reaction being carried out at an absolute pressure of from 2 to 200 mbar.

3. A process as claimed in claim 1 or 2, wherein the residence time of the reaction medium is set at from 1 to 60 minutes, based on the liquid reacted mixture.

4. A process as claimed in claim 1 or 2 or 3, wherein the products removed from the reactor are fed to a directly downstream rectification column for separating off the cleavage gases, gaseous or liquid product or product mixture being taken off by means of a further take-off point in addition to the top take-off, the one product being isocyanate-rich and the other product being rich in the hydroxyl component, depending on the order of the boiling points.

5. A process for the cleavage of a carbamic ester in a process as claimed in claim 1 or 2 or 3 or 4, wherein no solvent is added to the reaction medium.

6. A process for the cleavage of a carbamic ester in a process as claimed in claim 1 or 2 or 3 or 4 or 5, wherein the conversion of urethane into isocyanate is not less than 50% in one pass.

7. A process for the cleavage of a carbamic ester in a process as claimed in claim 1 or 3 or 4 or 5, wherein the pressure in the reactor is from 1 to 500 mbar.

8. A process as claimed in claim 4, wherein rectification is carried out at less than 500 mbar.

## Revendications

1. Procédé de scission thermique d'esters de l'acide carbamique monofonctionnels et polyfonctionnels en les isocyanates correspondants et le composant hydroxylé, en phase liquide, caractérisé en ce que l'on entreprend la réaction dans un réacteur qui comporte des chicanes pour l'établissement de la chaleur de réaction et dont la géométrie, caractérisée par la surface de dégazage par rapport au volume, permet la scission en un mélange biphasé à une teneur en gaz volumétrique supérieure à 50%.

2. Procédé suivant la revendication 1, caractérisé en ce que la vitesse du gaz qui se dégage à l'extrémité supérieure de la zone réactionnelle varie de 1 à 30 m/s, où l'on entreprend la réaction sous une pression absolue de 2 à 200 mbars.

3. Procédé suivant la revendication 1 ou 2, caractérisé en ce que l'on règle la durée de séjour du milieu de réaction à 1 à 60 min., par rapport à l'effluent liquide du réacteur.

4. Procédé suivant les revendications 1 à 3, caractérisé en ce que, en vue de la séparation des gaz de scission, on envoie les produits prélevés du réacteur dans une colonne de rectification raccordée immédiatement à la suite, où, en plus du prélèvement en tête, on soutire un produit ou un mélange de produits gazeux ou liquide, à l'aide d'un endroit de prélèvement supplémentaire, où en correspondance à la suite des points d'ébullition, l'un des produits est riche en isocyanate et l'autre produit est riche en composants hydroxylés.

5. Procédé de scission d'esters de l'acide carbamique dans un procédé suivant les revendications 1 à 4, caractérisé en ce que l'on n'ajoute pas de solvant au milieu de réaction.

6. Procédé de scission d'esters de l'acide carbamique dans un procédé suivant l'une quelconque des revendications 1 à 5, caractérisé en ce que la conversion atteint au moins 50% lors du passage de l'uréthanne en isocyanate.

7. Procédé de scission d'esters de l'acide carbamique dans un procédé suivant l'une quelconque des revendications 1 ou 3 à 5, caractérisé en ce que la pression dans le réacteur varie de 1 mbar à 500 mbars.

8. Procédé suivant la revendication 4, caractérisé en ce que l'on opère la rectification sous une pression inférieure à 500 mbars.
